# EUROPEAN PATENT SPECIFICATION

(11) **EP 2 616 569 B1**
(45) Date of publication and mention of the grant of the patent: **27.04.2022**
(21) Application number: 11760814.1
(22) Date of filing: 13.09.2011
(51) Int. Cl.: C01B 13/02, A61H 33/14, A61M 35/00, C25B 1/04, C25B 9/73, C01B 5/00

(54) **OXYGEN CONCENTRATOR AND METHOD**
SAUERSTOFFKONZENTRATOR UND VERFAHREN
CONCENTRATEUR D'OXYGÈNE ET PROCÉDÉ ASSOCIÉ

(30) Priority: 14.09.2010 GB 201015368; 13.09.2010 GB 201015265
(43) Date of publication of application: 24.07.2013
(73) Proprietor: Inotec AMD Limited, Waterbeach, Cambridge CB25 9PD (GB)
(72) Inventor: VINTON, Melvin Frederick, Fowlmere Cambridgeshire SG8 7SR (GB); FRAY, Derek John, Fowlmere Cambridge SG8 7SL (GB)
(74) Representative: Gill Jennings & Every LLP
(86) International application number: PCT/GB2011/001343
(87) International publication number: WO 2012/035298

(56) References cited:
- WO-A1-2008/138048
- WO-A2-2006/092612
- CA-A1- 1 198 710
- None

## Description

The invention relates to an oxygen concentrator and a method for concentrating oxygen from air, such as ambient air.

There is evidence in the literature that the supply of oxygen to a wound site can promote the healing of the wound. This applies to both humans and animals. This technique is know as topical oxygen therapy, and encourages the growth of fresh skin tissue to close and heal wounds.

In most instances, the affected limb is placed in a chamber (US 4,003,371 and US 3,744,491) or a bag (US 5,154,697 and US 5,478,310) and oxygen is fed to the chamber or bag from an oxygen cylinder. This is impractical for many patients as it restricts the mobility of the patient. To improve topical oxygen therapy, it would be desirable to have a more convenient supply of oxygen.

In an alternative approach, as described in US 5,578,022, US 5,788,682 and US 5,855,570, electrolytic devices may be incorporated into or above bandages that are placed over the wound. The cathode of the electrolytic device is exposed to air to form hydrogen peroxide that dissolves in a proton-conducting membrane adjacent the cathode. The hydrogen peroxide diffuses through the membrane to an anode where it is decomposed to form water and oxygen that is transmitted to the wound. The presence of hydrogen peroxide is a significant problem because it can kill healthy cells. It is therefore desirable to be able to produce pure oxygen for healing of wounds without the use of a hydrogen peroxide intermediate. Unfortunately, all known proton-conducting membranes are highly acidic, and under these conditions hydrogen peroxide is formed when oxygen is ionised at a cathode.

An alternative approach to electrolytic oxygen generation is to electrolyse water to produce hydrogen and oxygen, and to prevent oxygen from contacting the cathode. This avoids the production of hydrogen peroxide. WO 2006/092612 describes in one embodiment an electrolytic oxygen generator in which water is supplied from a source, or reservoir, of water to a proton-conducting membrane. The water is electrolysed between a cathode and an anode on either side of the membrane to form oxygen at the anode and hydrogen at the cathode. A catalytic apparatus is provided adjacent to the cathode in order to avoid releasing hydrogen from the oxygen generator into the atmosphere, and in order to decrease the consumption of water by the oxygen generation apparatus. The catalytic apparatus comprises a catalyst layer spaced from the cathode by a gas-permeable/liquid-permeable membrane. The catalyst layer is exposed to air. Hydrogen generated at the cathode diffuses through the gas-permeable/liquid-permeable membrane to the catalyst, where it reacts with atmospheric oxygen to form water. Thus, the hydrogen is not released into the atmosphere. At least some of the water diffuses back through the gas-permeable/liquid-permeable membrane to the cathode and the proton-conducting membrane, to supplement the water provided from the water source.

The inventors have tested an electrolytic oxygen generator of this type, implemented as a portable, ambulatory apparatus that can be worn by a patient. The oxygen supply from the oxygen generator is fed to a suitably-designed hyperbaric dressing covering a wound for treatment. In these tests, the oxygen generator has been powered by rechargeable batteries, and it has been found that the water source needs to be refilled significantly more often than the batteries need to be recharged.

It would be desirable for a portable oxygen generator to operate for as long as possible without needing either the batteries recharged or the water source replenished. The present invention aims to address this problem.

### Summary of Invention

The invention provides an oxygen concentrator, a method of concentrating oxygen, and an oxygen supply unit as defined in the appended claims, to which reference should now be made.

The invention provides an oxygen concentrator, rather than an oxygen generator. This reflects the fact that the apparatus according to the invention does not have, and does not need, a water reservoir.

In a first aspect, the invention provides an oxygen generator according to claim In use, a voltage is applied between the cathode and the anode, to electrolyse water in the proton-conducting membrane. The water is initially supplied through atmospheric humidity. Electrolysis produces oxygen at the anode and hydrogen at the cathode. The hydrogen passes through the diffusion layer (or permeable layer) spacing the catalyst from the cathode. At the catalyst, the hydrogen reacts with atmospheric oxygen within the cathode chamber, to produce water. The water passes back through the diffusion layer to the cathode and the proton-conducting membrane for further electrolysis. Surprisingly, the inventors have found that the oxygen concentrator can operate continuously, with no water supply other than atmospheric humidity, even at low humidity levels. In other words, the oxygen concentrator advantageously does not need a water reservoir and can electrolyse water derived only from humidity in the air and from the catalyst. As described above, electrolytic devices for concentrating oxygen from the atmosphere for topical oxygen therapy have been proposed, but in these prior art devices the cathode has been exposed to air to form hydrogen peroxide that dissolves in a proton-conducting membrane adjacent to the cathode. As described above, the creation of hydrogen peroxide is a significant problem. By contrast, the present invention provides an oxygen concentrator which operates by electrolysis of water, and in which the cathode is not exposed to atmospheric oxygen. The inventors have found that the provision of a catalyst apparatus positioned very close to or directly adjacent to the cathode converts the cathodically-generated hydrogen to water, and recycles the water to the proton-conducting membrane, with such little loss of water that the oxygen concentrator embodying the invention can operate without a water reservoir. This is completely contrary to the expectation and technical prejudice of the skilled person, who would expect a water reservoir to be necessary for the continuous generation of oxygen by electrolysis of water.

The performance of the oxygen concentrator is enhanced by the action of the ventilation means allowing air to flow to the catalyst, so that the cathodically-generated hydrogen can react with atmospheric oxygen.

In a preferred embodiment which may improve the performance of the oxygen concentrator still further, it is desirable that the ventilation means is arranged to control the flow of air into or through the cathode chamber in such a way that the ventilation of the cathode chamber is between predetermined upper and lower limits. The inventors have determined experimentally that a level of ventilation, or a level of air flow or air diffusion, between upper and lower limits enables enhanced operation of the concentrator. It is found that if ventilation of the cathode chamber falls below a predetermined lower limit or rises above a predetermined upper limit, the resistance of the electrolytic cell increases. But high efficiency of the oxygen concentrator may be continuously maintained if the ventilation of the cathode chamber is between predetermined upper and lower limits. Good electrical efficiency to produce a desired oxygen flow is desirable to enable an oxygen concentrator, such as a portable oxygen concentrator, to operate for as long as possible from a self-contained source of electrical power, such as a battery.

The electrochemical processes involved in the decrease in efficiency of operation outside the optimum range of ventilation are not fully understood. Nevertheless, the inventors believe that the operation of the oxygen concentrator is as follows.

If ventilation of the cathode chamber is less than a predetermined level, then it is believed that insufficient atmospheric oxygen reaches the catalyst to react with all of the hydrogen generated at the cathode. In that case, hydrogen may be released into the atmosphere and recycling of water from the catalyst to the proton-conducting membrane may be reduced. Consequently, the electrolysis of water may slow down and ultimately stop.

If the ventilation of the cathode chamber is above a predetermined level, then substantially all of the hydrogen generated at the cathode may react with atmospheric oxygen at the catalyst to produce water. However, some of the water may enter (or evaporate into) the air ventilating the cathode chamber and be carried away from the catalyst into the atmosphere. This reduces the amount of water recycled to the proton-conducting membrane, so that the rate of electrolysis may slow down. It is believed that this effect may vary depending on the humidity of the ambient air, or ambient atmosphere. If ambient humidity is very high, then the amount of water lost to the atmosphere may be reduced, and more of the water present as humidity in the atmosphere may be drawn into the diffusion layer and pass through the layer to the proton conducting membrane. By contrast, if ambient humidity is very low, then more water may be lost from the catalyst into the atmosphere, and less humidity may be absorbed into the diffusion layer and passed to the proton-conducting membrane.

Within the desired range of ventilation of the cathode chamber, sufficient atmospheric oxygen may reach the catalyst to react with substantially all of the hydrogen produced at the cathode, and an advantageously large proportion of the water may pass back through the diffusion layer to the proton-conducting membrane for further electrolysis. Preferably none, or only a very small fraction, of the water may be lost into the air ventilating the cathode chamber.

It is believed that the catalytic apparatus may operate by virtue of diffusion gradients across its thickness. Because hydrogen is converted to water at the catalyst, hydrogen produced at the cathode may diffuse down a diffusion gradient from the cathode to the catalyst, and because water is electrolysed at the proton-conducting membrane, water produced at the catalyst may diffuse down a diffusion gradient towards the proton-conducting membrane. The ventilation of the cathode chamber should therefore be sufficiently high to allow reaction of substantially all of the hydrogen reaching the catalyst, and should be sufficiently low that water generated at the catalyst tends to diffuse towards the proton-conducting membrane rather than being lost into the atmosphere.

The performance of the oxygen concentrator might be expected to vary depending on the humidity of the ambient air. In practice, it has been found by the inventors that the oxygen generated by the oxygen concentrator is at substantially the same humidity as the ambient air. It is therefore understood that the combination of ambient humidity entering the cathode chamber and the efficient recycling of water by the catalytic apparatus are sufficient to enable continuous oxygen generation at a very wide range of ambient humidity. The concentrator may not be able to operate at extremely low ambient humidity (for example in tests of one oxygen concentrator embodying the invention, the inventors obtained satisfactory operation down to 37% relative humidity, though design improvements may allow operation at lower relative humidity, such as 25% or even 10%), but it may be noted that an advantageous application of the concentrator is to produce oxygen in an ambulatory apparatus for topical oxygen therapy. The inventors have found that if the oxygen concentrator is worn within clothing, then the proximity to the wearer's body means that the humidity may be sufficient to allow continuous oxygen generation at a predetermined flow rate even if the ambient humidity level outside the clothing is disadvantageously low.

Advantageously, because the oxygen concentrator operates by electrolysing water and prevents the access of atmospheric oxygen to the cathode (by virtue of the presence of the catalytic apparatus), hydrogen peroxide is not formed.

The oxygen concentrator may in principle be of substantially any size but its use is particularly advantageous in a portable device. In a portable, or ambulatory, device the elimination of the need to provide and replenish a water reservoir means that the concentrator can operate advantageously for an extended period of time. The period of operation is limited only by the power source and it has been found that a conveniently-sized portable unit using rechargeable batteries can operate for a week or more before the batteries need to be recharged.

A portable oxygen concentrator may comprise a proton-conducting membrane having an active area, between the cathode and the anode, of between 150mm² and 2000mm², preferably between 300mm² and 1000mm², and particularly preferably between 400mm² and 600mm². For example, the active area of the proton-conducting membrane may be a circle of 25mm diameter. (The areas and shapes of the anode, cathode, diffusion layer and catalyst may advantageously all be the same as the active area and shape of the membrane.) Such a cell operating at a voltage of about 1.2V and a current of 60mA can produce oxygen continuously at about 15ml/hr. This is a convenient rate of oxygen supply for provision to a suitable hyperbaric dressing covering a wound.

The proton-conducting membrane, the cathode, the anode, the catalyst and the diffusion layer may all be in the form of planar layers, in contact with each other to form a stack of layers. Advantageously, the cathode and the anode may be in the form of layers coated on the first and second sides of the proton-conducting membrane to form a Membrane Electrode Assembly (MEA), and the catalyst may be in the form of a layer coated onto one side of the diffusion layer. In the assembled oxygen concentrator, the diffusion layer may then be pressed into contact with the cathode.

It is necessary to provide a voltage between the cathode and the anode. In a preferred embodiment, this may be achieved by placing the stack of layers described above between a cathode-side conducting sheet and an anode-side conducting sheet, to which a supply of electricity can be connected. In this embodiment, the diffusion layer must be electrically conducting in order to carry the electric current from the cathode-side conducting sheet to the cathode.

The cathode-side conducting sheet should be porous or perforated in order to allow air to flow to the catalyst. Similarly, the anode-side conducting sheet should be porous or perforated in order to allow oxygen to flow away from the anode. Both conducting sheets may conveniently be implemented as perforated or foraminous metal sheets. These should be corrosion resistant, for example stainless steel sheets.

Advantageously the cathode-side and anode-side conducting sheets may be pressed against the opposite sides of the stack of layers discussed above, and may be of a predetermined area and shape corresponding to the active area and shape of the proton-conducting membrane. The internal cross-sectional area and shape of the cathode chamber may advantageously be substantially the same as the area and shape of the active portion of the membrane (with clearance provided so that the catalytic apparatus and other components can fit into the cathode chamber). The depth of the cathode chamber, measured perpendicular to the membrane, should be sufficient to accommodate the catalytic apparatus and any cathode-side conductive sheet, and to allow sufficient ventilation such that air can flow to the catalyst.

The cathode chamber may therefore have a depth greater than 0.4mm, 0.6mm 1mm or 2mm, and less than 10mm, 7mm or 5mm.

Hydrogen is generated across the entire active area of the proton-exchange membrane, and may diffuse directly through the diffusion layer from the cathode to the catalyst. Therefore, it is important that the entire area of the catalyst should be ventilated.

In order to allow this, the ventilation means comprise one or more vents defined through a wall of the housing of the cathode chamber. The level of ventilation in the cathode chamber may be related to the total area of the vent or vents. For a typical oxygen concentrator embodying the invention, the total area of the vent or vents may be between 7mm² and 80mm², preferably between 10mm² and 40mm² and particularly preferably between 12mm² and 20mm².

In addition to the provision of a vent or vents, the cathode chamber may be filled with a porous material, such as a foam or a sintered material. This may advantageously allow air to flow or diffuse through the cathode chamber but may act as a baffle to moderate the ventilation to prevent the bulk flow of air, or draughts of air, through the cathode chamber.

As described above, the oxygen concentrator may be implemented in a range of different sizes. Thus, the ventilation means may alternatively be defined in terms of the size of the oxygen concentrator and, in particular, the active area of the proton-conducting membrane. Thus, the active area of the membrane may be between 10 and 70 times, preferably between 25 and 55 times, and particularly preferably between 30 and 45 times the total area of the vent or vents.

The ventilation of the cathode chamber may be affected by its shape. Thus, for advantageous performance of the ventilation means, the depth of the cathode chamber may be defined as being proportional to a lateral dimension, or a maximum lateral dimension, of the cathode chamber. For example, the cathode chamber may have a lateral dimension which is between 10 and 70 times its depth or preferably between 25 and 50 times its depth.

In a preferred embodiment, in order to achieve ventilation across the entire area of the catalyst, the ventilation means may be distributed across a lateral dimension of the cathode chamber. For example the ventilation means may be in the form of two or more vents spaced at different positions in a wall of the housing.

The inventors have found that optimum operation of the oxygen concentrator is preferably achieved at an applied voltage of between 0.75V and 2V, more preferably between 1V and 1.5V, and particularly preferably about 1.2V. These are voltages applied to the cell, for example between cathode-side and anode-side conducting sheets as described above. The cell potential measured between the cathode and the anode may be less than the applied cell voltage but the inventors have measured cell efficiencies of more than 80%, or more than 90%, and so it is believed that voltage losses within the cell are small.

Corresponding to the voltages described above, the oxygen concentrator may advantageously operate at a current density over the active area of the proton-conducting membrane, of between 50Am⁻² and 250Am⁻², preferably between 75Am⁻² and 200Am⁻², and particularly preferably between 100AM⁻² and 150Am⁻².

It has been found that these electrical conditions may advantageously produce a flow of oxygen gas from the anode of up to 30ml/hour (measured at atmospheric or ambient pressure) for each 500mm² of the area of the anode.

In practice, for topical oxygen therapy, in a preferred embodiment oxygen may be produced at a slightly hyperbaric pressure, for example at about 50mbar above atmospheric pressure. In more general terms, the anode chamber may operate at a different pressure from the cathode chamber. The anode chamber and the cathode chamber should therefore be hermetically isolated from each other, so as to prevent flow of gas between them. This provides the further advantage of preventing oxygen generated at the anode from contacting the cathode, which could disadvantageously lead to formation of hydrogen peroxide.

In order to enable operation without a water reservoir, the efficiency of the oxygen concentrator is preferably as high as possible, both in terms of electrical performance and in terms of water recycling from the catalyst to the proton-conducting membrane. In a preferred embodiment, the structure of the oxygen concentrator may therefore be as follows. The cathode and/or the anode may be in the form of layers coated onto opposite sides of the proton-conducting membrane to form a MEA. The catalyst may be in the form of a layer coated onto the diffusion layer. Advantageously, the diffusion layer of the catalytic apparatus may then be pressed against the cathode. In other words, the catalyst, the diffusion layer, the cathode, the proton-conducting membrane and the anode may advantageously be in the form of layers pressed together. This may advantageously position the catalyst as close as possible to, but separated from, the cathode.

The layers may be pressed together between a cathode-side conducting sheet and an anode-side conducting sheet for the supply of electric current to the layers. The inventors have found that a high pressure urging the layers together may advantageously be used. For example, a pressure of more than 0.5MPa, preferably more than 0.8MPa, and particularly preferably more than 0.9MPa may be applied to urge the conducting sheets towards each other and to compress the layers of the cell together. This means, for example, that the layers of a cell in which the active area of the proton-conducting membrane is about 500mm² should particularly preferably be pressed together with a force of about 450 Newtons. In the inventor's experiments, it has been found that at lower pressures, the electrical resistance of the cell is increased, while at higher pressures little further decrease in electrical resistance is obtained.

The layers may be loaded or pressed together in any convenient manner. For example, an oxygen concentrator may conveniently be housed within a rigid housing and resilient elements positioned between the housing and the stack of layers to apply the required pressure. Suitable resilient elements may comprise O-rings or elastomeric foams, such as polyurethane foams. Electrically-conductive foams may be used.

In the construction of an oxygen concentrator it is important that the materials used should be sufficiently corrosion resistant.

To generate oxygen, the oxygen concentrator should be coupled to a suitable electrical power supply. In a preferred embodiment, the oxygen concentrator may be operated in a current-controlled mode. The applied current corresponds to the rate of oxygen generation at the anode and so a user may set the power supply to apply a predetermined constant current to the cell depending on the desired oxygen flow rate. A corresponding voltage is then required to drive the selected current. If, in use, the oxygen concentrator fails to operate normally, its electrical resistance will tend to rise, so that the required voltage to drive the selected current will also rise. In a preferred embodiment, the power supply is arranged so that if the required voltage rises above a predetermined voltage threshold, the power supply switches to a stand-by mode, in which the applied voltage is greatly reduced or switched off.

As described above, a failure mode of the cell may be if the ambient atmosphere is of extremely low humidity. In that case, the water content of the proton-conducting membrane may progressively reduce and the resistance of the cell may rise.

A further aspect of the invention may thus provide an oxygen supply unit comprising an oxygen concentrator, a battery and a power supply for providing a predetermined current, for example a user-selected current, to the oxygen concentrator. The power supply may therefore be a constant-current power supply. The oxygen supply unit may be portable, or wearable, for example for use for ambulatory topical oxygen therapy.

As described above, an oxygen concentrator embodying the invention advantageously comprises a cathode chamber having a ventilation means which controls the flow or diffusion of air from the atmosphere to the catalyst such that the concentrator can produce a continuous flow of oxygen at a wide range of ambient humidity. As noted above, the inventors' experiments have demonstrated the continuous generation of oxygen down to 37% relative humidity, or even 35% relative humidity. As also described in this document, the ventilation means should provide a level of ventilation between upper and lower limits, and if the ventilation of the cathode chamber falls below a predetermined lower limit or rises above a predetermined upper limit, the electrical resistance of the electrolytic cell may disadvantageously increase. In more general terms, this means that the resistance per unit area of the cell (the area of the catalyst or of the Membrane Electrode Assembly) may increase.

It is usually desirable to operate an oxygen concentrator so as to generate a constant flow of oxygen at a desired flow rate. The oxygen flow rate is related to the current flowing through a cell and so it is commonly desirable to operate a cell at a predetermined constant current. In this case, if the cell resistance rises, a rise in the voltage applied to the cell is observed. If the resistance of the cell increases, initially the cell may continue to operate but at reduced efficiency, and if the resistance rises too far, the oxygen output falls and eventually the MEA may be damaged unless the applied current is reduced.

The electrical performance of an oxygen concentrator at a known relative humidity level may thus provide a means for assessing whether or not the level of ventilation at the catalyst is between the upper and lower ventilation limits. In an oxygen concentrator embodying this aspect of the invention, the optimisation of the ventilation of the catalyst as described above may advantageously increase, or maximise, the flow rate of oxygen that can be produced continuously. As a result, the inventors have found that oxygen concentrators having the following performance have ventilation means that achieves ventilation of the catalyst between the upper and lower limits identified above.

For such a cell in air of relative humidity equal to or greater than 35%, a constant current density can be applied such that, after any initial voltage transient, the cell produces a continuous oxygen flow equal to or greater than 24 litres per hour per m² of the area of the catalyst (or of the cathode or MEA) at NTP (normal temperature and pressure, 25 C and 1 atmosphere). For experimental purposes a continuous oxygen flow may be considered to be a flow maintained for 3 hours or more. An oxygen concentrator achieving this performance must meet the requirement that the ventilation of the catalyst is between the upper and lower limits described above with reference to embodiments of the present invention. If the ventilation were above the upper limit then excessive water would be lost to the atmosphere, and if the ventilation were below the lower limit then insufficient atmospheric oxygen may reach the catalyst to convert to water all of the H₂ generated at the cathode. In either case the performance of the cell would decrease over time until the oxygen flow falls below the threshold level of 24 l/hr/m⁻². At the same time, the cell resistance would increase and the voltage applied to the cell would correspondingly increase. An alternative assessment of an unacceptable decrease in cell performance may thus be made with reference to the increase in voltage required to drive the predetermined constant current (after any initial voltage transient period when the cell is switched on, which typically lasts only 30 seconds or 1 minute at most). For example an increase of the operating voltage of 20%, or 30%, for example within the first 10 minutes or 20 minutes of operation, may be considered unacceptable. Such an increase in operating voltage may therefore indicate that the ventilation of the catalyst is outside the upper and lower limits described above.

The value of the current density required to achieve the oxygen flow equal to or greater than 24 l/hr per m² of the catalyst (or of the cathode or MEA), and the voltage applied to the cell to achieve that current density, may vary depending on factors other than the ventilation of the catalyst, such as the electrical resistance of the cell and the thickness of components such as the proton-conducting membrane. But in a cell having low electrical resistance and an advantageously thin proton-conducting membrane, and in which the distance between the catalyst and the cathode is advantageously small (as described in the specific embodiments of the invention hereinafter), then the minimum constant current density to generate an oxygen flow of 24 l/hr/m² at NTP is about 110Am⁻² (based on the area of the cathode or the MEA, or of the catalyst). This corresponds to a constant current of about 55mA applied to a cell having a circular catalyst of 25mm diameter (490mm²) producing at least about 12ml/hr of oxygen at NTP.

As the skilled person would be aware, the Faraday equation enables calculation of a maximum number of moles per unit time of oxygen gas, and therefore a maximum oxygen flow rate at a given temperature and pressure, such as at NTP, that can be produced by a given current flowing through an oxygen concentrator. The generation of 24 l/hr/m² at 110Am² described above is above about 95% of the maximum oxygen flow at this current, and so such a cell may be considered to be more than 95% efficient. Oxygen concentrators having ventilation of the catalyst within the desired range described above may produce a continuous flow of oxygen at more than 85%, preferably more than 90%, and particularly preferably more than 92% or 95% efficiency.

Preferably, this performance is achieved at a cell voltage of between 0.8V and 1.2V, or about 1.0V.

In a cell of this type, other metrics may also be used to assess whether the ventilation of the catalyst is within the prescribed range. For example the multiple of voltage times the catalyst area (or the cathode or active MEA area), should not rise above 6V.cm², and/or the value of the voltage divided by the current times the catalyst area (or the cathode or active MEA area) should not rise above 120 Ohm.cm².

A cell having the preferred ventilation of the catalyst described above may be able to produce a constant oxygen flow at a lower relative humidity than mentioned above, such as at less than 37% or 35% relative humidity, but the flow may be less than 24 l/hr/m² and the cell may only be able to operate continuously at a reduced current.

The invention has been described in the context of topical oxygen therapy, but as the skilled person would appreciate it may be used in any application where a suitable flow of pure oxygen is required.

Specific embodiments of the invention will now be described by way of example, with reference to the accompanying drawings, in which:
Figure 1 is an exploded view of an oxygen concentrator according to a first embodiment of the invention;
Figure 2 is a three-quarter view of a cell base of the oxygen concentrator of Figure 1;
Figure 3 is a plan view, from beneath, of the cell base;
Figure 4 is a three-quarter view of a cell cover of the oxygen concentrator of Figure 1;
Figure 5 is a plan view, from beneath, of the cell cover;
Figure 6 is a partial exploded view of the oxygen concentrator of Figure 1, showing transverse sections of the cell base, the cell cover, an O-ring and a MEA oriented for assembly;
Figures 7 and 8 are plan and side views of resilient polyurethane foam layers of the oxygen concentrator of Figure 1;
Figure 9 is a plan view of a cathode-side conductive layer of the oxygen concentrator of Figure 1;
Figure 10 is an enlarged partial view of the conductive layer of Figure 9;
Figure 11 is a plan view of the membrane electrode assembly (MEA) of the oxygen concentrator of Figure 1;
Figure 12 is an exploded view of an oxygen concentrator according to a second embodiment of the invention;
Figure 13 is a three-quarter view of the oxygen concentrator of Figure 12, in assembled form;
Figure 14 is a transverse section of the assembled oxygen concentrator of Figure 13;
Figure 15 is a diagrammatic section of a portion of the membrane electrode assembly (MEA) and regeneration catalyst of an oxygen concentrator showing the electrochemical processes of the cell; and
Figure 16 is a schematic diagram of an oxygen supply unit embodying the invention.

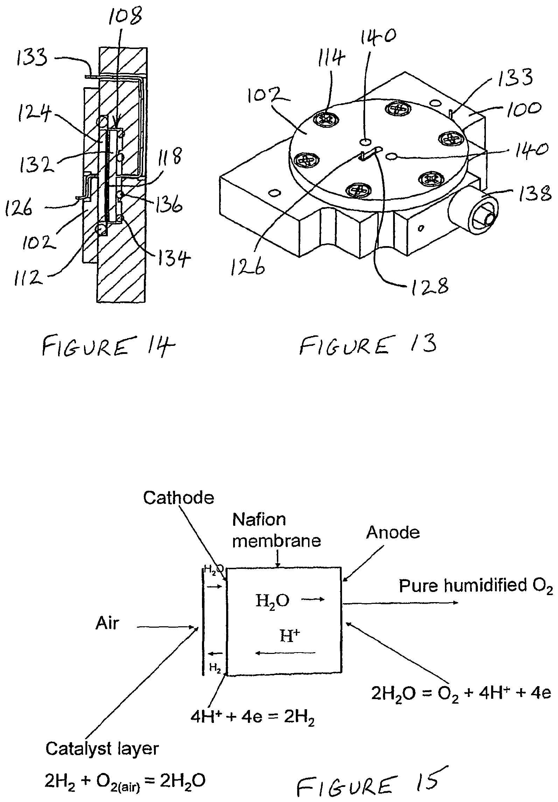

Figure 15 illustrates the electrochemical processes in the cell of an oxygen concentrator. It shows a diagrammatic section of part of the proton-conducting membrane which is in this case a Nafion (RTM) membrane. An anode and a cathode are coated onto opposite sides of the membrane and, on the cathode side, a regeneration catalyst layer is spaced from the cathode by a diffusion layer. Water in the membrane reacts at the anode to produce gaseous oxygen and hydrogen ions. The hydrogen ions diffuse through the membrane to the cathode where they combine with electrons to form gaseous hydrogen. The hydrogen diffuses through the diffusion layer to the catalyst layer, where it reacts with atmospheric oxygen to form water. The water diffuses back through the diffusion layer to the cathode and the Nafion membrane. The water then diffuses through the membrane to the anode for re-electrolysis.

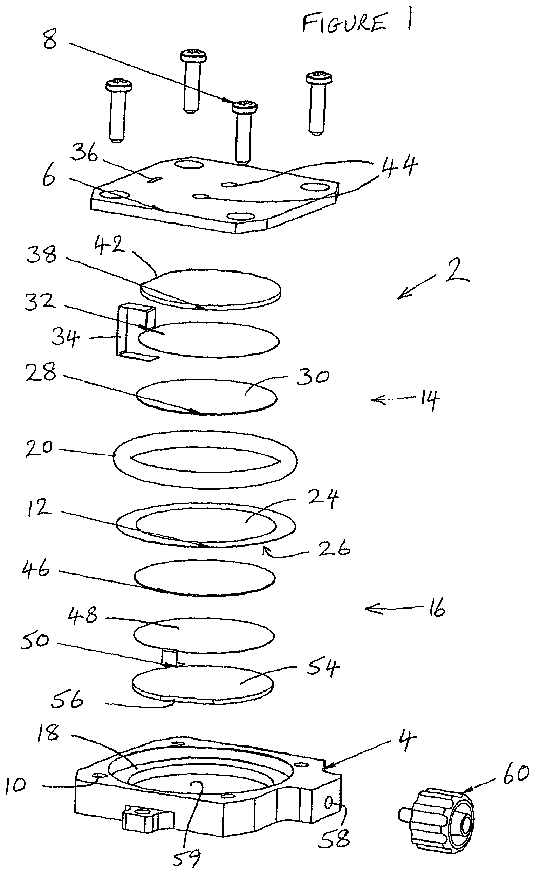

Figure 1 is an exploded view of an oxygen concentrator 2 according to a first embodiment of the invention. The concentrator is constructed as a stack of substantially circular layers (as described below) housed between a cell base 4 and a cell cover 6. The cell base and the cell cover are machined from polymethyl methacrylate, but could be fabricated from any suitable, inert material. In the assembled concentrator the cover is secured to the base by four bolts 8 which pass through holes in the cover and screw into threaded holes 10 in the base.

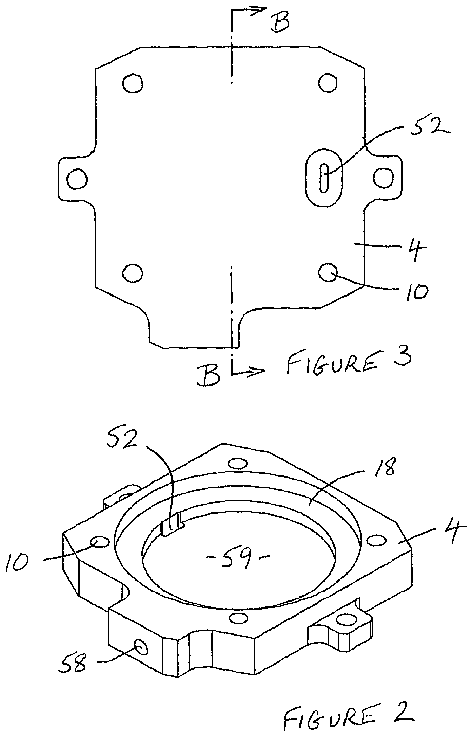

Figures 2, 3, 4 and 5 show three-quarter and plan views of the cell base and the cell cover.

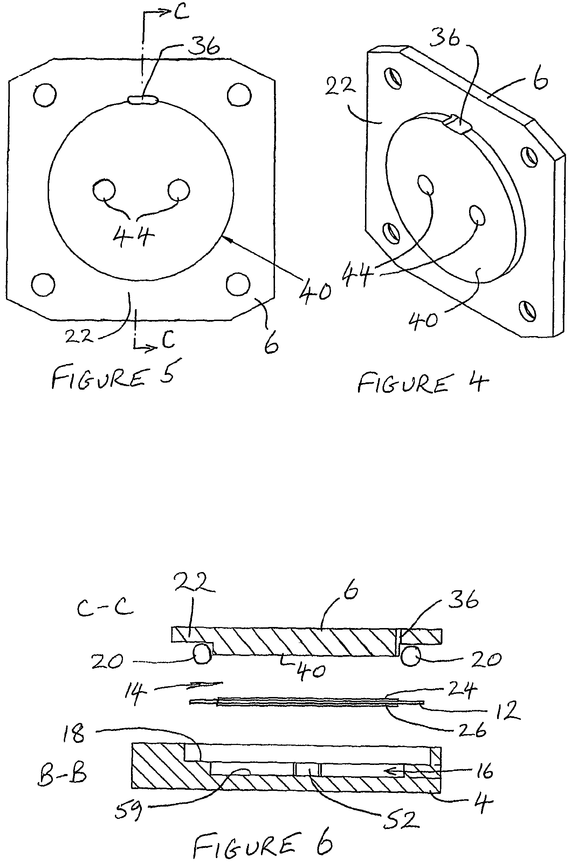

When the oxygen concentrator is assembled, a proton-conducting membrane 12 divides the space within the cell into a cathode compartment 14 and an anode compartment 16, as illustrated in Figure 6. The membrane is of Nafion (RTM), type NRE212. The membrane is circular, of 32mm outside diameter, and seats on a corresponding-sized step 18 of the cell base.

A radially-outer portion of the membrane is seated on the step 18 and is urged against the step in the assembled cell by a nitrile O-ring 20. The O-ring is urged against the membrane by a radially-outer flange portion 22 of the cell cover and forms gas- and liquid-tight seals between the radially-outer portion of the membrane and the step 18 of the cell base, between the radially-outer portion of the membrane and the O-ring, and between the O-ring and the cell cover, to define the cathode chamber 14 and the anode chamber 16.

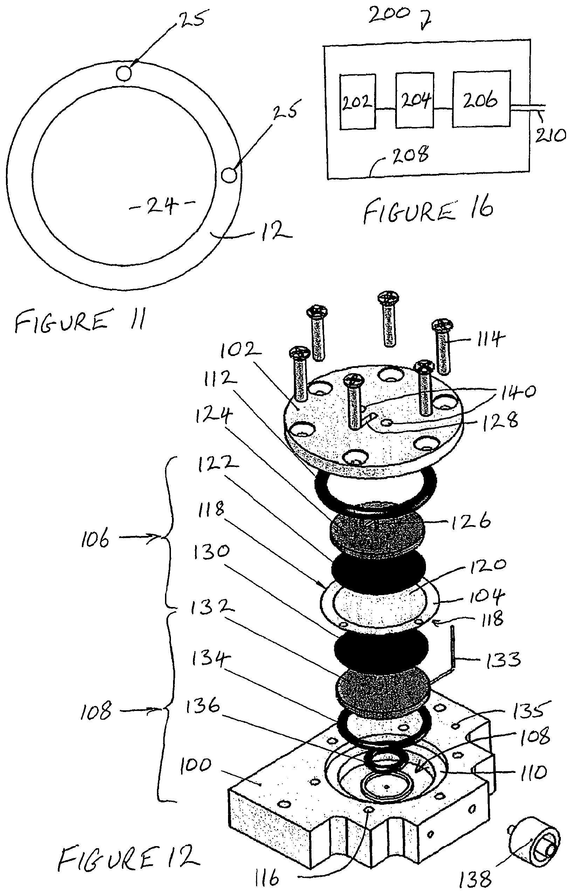

A cathode 24 and an anode 26 are coated on opposite sides of a central portion of the membrane to form a MEA. Each of the cathode and the anode are in the form of a 25mm circle, centrally disposed on the circular membrane as shown in Figure 11. The thickness of the Nafion membrane is approximately 0.14mm. The cathode and the anode are sputtered. The cathode loading is at least 0.3mg of platinum per cm² and the anode loading is at least 1mg of iridium per cm². The MEA is marked with coloured dots 25 to identify the anode and cathode sides.

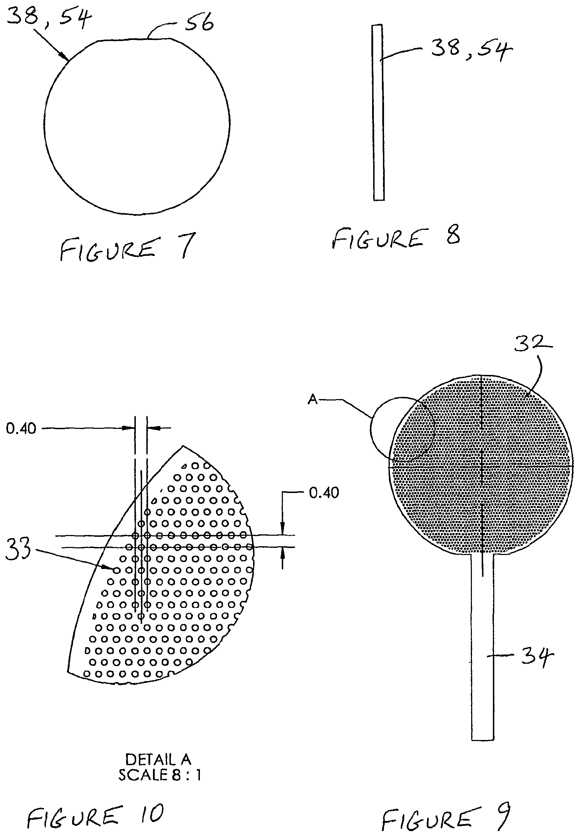

On the cathode side of the membrane, the cathode chamber contains a stack of three components (see Figure 1). Adjacent the cathode is a diffusion layer 28 coated on its side spaced from the cathode with a sputtered platinum catalyst layer 30. The diffusion layer may be of any suitable porous cloth, paper or composite (e.g. plastic composites containing carbon, graphite, carbon nanotubes or some combination thereof). It is electrically conductive and is optionally hydrophobic. In the embodiment, the diffusion layer comprises a 200µm non-organic woven 10% PTFE carbon layer. The catalyst may be any suitable catalyst, or catalyst plus support material, for adequately catalysing the reaction of hydrogen and oxygen to form water. In the embodiment, the catalyst is a sputtered layer of Pt, at a density of 2mg.cm⁻². The diffusion layer and catalyst are in the form of a 25mm disc, corresponding to the diameter of the anode and the cathode, which define the active area of the MEA. Adjacent the catalyst is a conductive layer in the form of a cathode-side perforated stainless steel disc 32. This is illustrated in plan view in Figure 9 and on an enlarged scale in Figure 10, to show that an array of small circular perforations 33 is formed through the disc. The perforations are 0.2mm in diameter and arranged in a close-packed array at 0.4mm spacing. In the assembled cell, the perforations allow access of air to the catalyst 30. A tab 34 extends from the edge of the stainless steel disc. In the assembled cell this emerges through a corresponding slot 36 in the cell cover for the connection of an electrical supply.

Finally, a disc of resilient, compressible, porous polyurethane foam 38, again of 25mm diameter, is positioned between the stainless steel disc and a raised circular central portion 40 of the cell cover, again of 25mm diameter. The foam disc is illustrated in Figures 7 and 8 and, before compression, is approximately 1.4mm in thickness. A portion of its edge 42 is cut away to allow passage of the tab 34 of the stainless steel disc. When the cell is assembled, the polyurethane foam disc is in a compressed state, in order to press the layers of the cell together. The polyurethane foam is porous, in order to allow access of air to the catalyst. The foam may be conductive, for example being a metallized, nickel over copper plated, polyurethane foam.

Two circular holes 44 are formed through the cell cover, through its thicker central portion 40, so as to open into the cathode chamber. These holes provide ventilation for the cathode chamber, and allow access of air, through the polyurethane foam and the perforations of the stainless steel disc, to the catalyst 30.

In the anode chamber, a conductive, gas-permeable membrane 46 is positioned adjacent to the anode. This membrane is in the form of a 25mm disc, corresponding to the shape and size of the anode. The material of the membrane may be the same as for the diffusion layer 28 described above. An anode-side conductive layer, in the form of a further perforated stainless steel disc 48, contacts the side of the gas-permeable membrane 46 opposite to the anode. The perforations are the same as for the cathode-side stainless steel disc described above. A tab 50 extends from an edge of the stainless steel disc and emerges through a corresponding slot 52 in the cell base for the connection of an electricity supply.

If desired, the membrane 46 may be omitted as long as adequate electrical contact is then maintained between the anode and the anode-side stainless-steel disc.

A resilient, compressible layer 54 of polyurethane foam is positioned between the stainless steel disc and a central, recessed circular portion 59 of the cell base. A small portion 56 of the circular outer edge of the foam disc 54 is cut away to allow the tab 50 of the stainless steel disc to pass through the slot 52 in the cell base. The foam may optionally be electrically conductive.

A gas outlet passage 58 leads radially outwardly from the anode chamber 16 and terminates at a luer 60, which allows the oxygen outlet to be coupled to a tube for connection to, for example, a hyperbaric dressing covering a wound.

When the oxygen concentrator is assembled, the various layers described above are placed in a stack between the cell base and the cell cover. The screws 8 are then engaged with the cell base and, as the screws are tightened, the resilient foam layers 38, 54 are compressed to apply a force to urge the layers of the stack together. In addition, tightening the screws compresses the O-ring 20 to seal the outer periphery of the cell and to separate the cathode chamber from the anode chamber. The thickness and compressibility of the foam layers, and the spacing between the raised central portion 40 of the cell cover and the central circular portion 59 of the cell base, are predetermined such that a force of between 440 and 450 Newtons is applied to the layers between the foam discs. This ensures good electrical contact between the layers to minimise the electrical resistance of the cell, and ensures close contact between the diffusion layer supporting the catalyst and the adjacent cathode and proton-conducting layer. The resilience of the foam layers also advantageously accommodates any small variations in the dimensions of the components of the oxygen concentrator, for example due to manufacturing tolerances.

Before assembly of the cell, each foam layer has a thickness of about 1.4mm. In the assembled cell the cathode chamber, containing one compressed layer of foam, has a depth of about 0.7mm.

In an alternative embodiment, only one of the two foam layers may be needed to load the stack of layers in the cell together.

In use, an electricity supply is connected to the tabs 34, 50 of the stainless steel discs and a current of approximately 60mA is passed through the cell. The oxygen concentrator is positioned so that the external openings of the ventilation holes 44 in the cell cover are open to the atmosphere. Under these conditions, approximately 15ml/hr of oxygen gas is generated at the anode and emerges through the luer 60, at a pressure of approximately 50mbar above atmospheric pressure. A voltage of approximately 1.2V is required to drive the 60mA current. This is a suitable voltage to obtain an advantageously long battery life from a rechargeable battery.

The inventors have tested this cell at voltages above 1V and at a range of currents up to 120mA and found that, within this range, oxygen is continually generated at the anode at a rate proportional to the current. It is possible that higher currents could be applied in order to generate higher rates of oxygen flow. It is anticipated, however, that at high applied voltages, problems of corrosion may arise within the cell.

The power supply for the cell is designed so that if the cell resistance increases, so that the voltage required to drive the predetermined or preselected current rises unacceptably, then the power supply switches to a stand-by mode. In a preferred embodiment the power supply switches off if the voltage required by the cell rises above 2V. For example, in tests of the oxygen concentrator it has been found that this may happen if one of the vents 44 accidentally becomes blocked, so that ventilation of the cathode chamber is reduced.

To investigate the performance of the cell of this embodiment, and in particular the effect of ventilation of the cathode chamber, cells of the same size and geometry to that shown in Figures 1 to 11 were made, but having ventilation means comprising different numbers of similar ventilation holes 44 in the cell cover. The two ventilation holes 44 shown in Figure 1 have a total area of 13.2mm². A similar cell was made having one ventilation hole, of area 6.6mm², and a similar cell was made having four ventilation holes, of total area 26.4mm². Each cell was supplied with a constant current of 55mA at a temperature of 22 (±3)°C, relative humidity of 46 (+10/-5)% and at atmospheric pressure of 1006 (±10) mb. For the cell having two ventilation holes, oxygen was continuously produced (after a start-up transient lasting less than one minute) at between 9 and 11ml/hr for the duration of the test, 30 hours. The cell voltage was stable at about 1V. For the cells having one ventilation hole or four ventilation holes, the voltage required to drive the 55mA current steadily rose over the first 20 minutes of operation to above 1.5V, at which point the tests were terminated to prevent damage to the cells. These test results illustrate the inventors' understanding that the level of ventilation, or of access of the catalyst to the atmosphere, is critical. Too little and the catalyst is starved of atmospheric oxygen and fails to recycle enough water to maintain humidity in the MEA. Too much and the water is lost from the cell. In both cases an increase in electrical resistance results due to the MEA reducing in conductivity.

Figure 12 is an exploded view of an oxygen concentrator according to a second embodiment of the invention. Figure 13 shows a three-quarter view of the assembled cell and Figure 14 shows the assembled cell in transverse section. In the same way as in the first embodiment, the oxygen concentrator comprises a stack of layers housed between a cell base 100 and a cell cover 102. Each of the layers is circular, of 25mm diameter, except for a proton-conducting membrane 104 which is of 32mm diameter.

As in the first embodiment, the membrane 104 divides the cell into a cathode chamber 106 and an anode chamber 108. The anode chamber is contained within a blind cylindrical recess formed in the cell base. The cylindrical recess is encircled by a step 110 on which a radially-outer portion of the proton-conducting membrane seats. A nitrile O-ring 112 of 26.5mm internal diameter and 3mm section is positioned on top of the membrane and is compressed against the membrane by the cell cover 102. Thus, when the cell cover is in position on the cell base, the radially-outer portion of the proton-conducting membrane 104 seals against the step 110 in the cell base, and the O-ring 112 forms a seal between the membrane and the cell cover.

In the assembled cell, the cell cover is held in position on the cell base by six screws 114 received in corresponding threaded holes 116 in the base.

As in the first embodiment, the proton-conducting membrane is a Nafion membrane. A platinum anode 118 and a platinum cathode 120 are sputtered onto opposite sides of a central circular portion of the membrane, of 25mm diameter. This forms a Membrane Electrode Assembly (MEA) and defines the working area of the membrane.

In the assembled cell, an electrically-conductive diffusion layer 122 is positioned adjacent to and in contact with the cathode. A platinum catalyst layer is sputtered onto the face distant from the cathode. A porous metal disc 124 formed of sintered metal particles, of AISL 316L stainless steel, is positioned between the catalyst and the cell cover. A metal wire 126 is connected to a central point of the sintered disc and emerges through an opening 128 in the cell cover for connection to an electricity supply.

Adjacent to the anode is positioned an anode-side diffusion layer, for example a gas-permeable membrane, 130, which allows the passage of oxygen away from the anode. An anode-side sintered metal disc 132 is positioned adjacent to the diffusion layer, within the anode chamber 108, and seats on two concentrically-arranged O-rings 134, 136 seated on a base of the anode chamber formed in the cell base. The sintered disc is of AISL 316L stainless steel. The O-rings are respectively of 21mm and 8mm internal diameter, and both are of 2mm section. When the cell is assembled, the two O-rings are resiliently compressed and so exert a force on the anode-side sintered disc 132 which is substantially rigid. This in turn presses the layers of the cell structure together, to ensure good electrical contact between the layers. As in the first embodiment, it is found that a compressive force of between 400N and 450N is desirable.

A wire 133 is connected to a central portion of the anode-side sintered disc and emerges though a hole 135 in the cell base, for connection to an electricity supply.

As in the first embodiment, a hole is defined between the anode chamber and an external surface of the cell base for connection to a luer fitting 138.

The oxygen concentrator of the second embodiment is suitable for connection to a power supply as described in relation to the first embodiment, and its performance is similar to the oxygen concentrator of the first embodiment.

Two 2.9mm diameter holes 140 are formed through the cell cover to ventilate the cathode chamber as described above. The holes are spaced on opposite sides of the centre of the circular cathode chamber, between the centre and the outside diameter of the circular cathode chamber, to ensure even ventilation of the cathode chamber across the entire area of the catalyst. Ventilation occurs through the porous metal sinter, which acts as a baffle to prevent bulk airflow, or draughts, through the cathode chamber as described above.

Figure 16 is a schematic diagram of an oxygen supply unit 200 embodying the invention. A rechargeable battery 202 is connected, through a power supply unit 204, to an oxygen concentrator 206. The battery, the power supply unit and the oxygen concentrator are housed in a portable, wearable housing 208. An oxygen outlet 210 is mounted on an outer surface of the housing for connection, for example, to a tube leading to a hyperbaric dressing.

## Claims

1. An oxygen concentrator (2) comprising;
a proton-conducting membrane (12);
a cathode (24) contacting a first side of the membrane;
an anode (26) contacting a second side of the membrane;
a catalytic apparatus comprising a catalyst (30) and a diffusion layer (28), the diffusion layer spacing the catalyst from the cathode; and
a housing (4, 6) defining a cathode chamber (14), the catalytic apparatus being contained within the cathode chamber and the housing defining one or more vents (44) enabling air to flow to the catalyst,
wherein the oxygen concentrator does not have a water reservoir and is configured to sustain its operation by electrolysing water derived only from humidity in the air and the catalyst and by provision of the catalytic apparatus positioned very close to or directly adjacent to the cathode such that the catalytic apparatus converts cathode-generated hydrogen to water and recycles the water to the proton-conducting membrane with little water loss, and
wherein the cathode is not exposed to atmospheric oxygen.

2. An oxygen concentrator according to claim 1, wherein the one or more vents (44) enable ventilation of the cathode chamber (14) below a predetermined upper limit of ventilation rate and above a predetermined lower limit of ventilation rate;
optionally wherein ventilation above the predetermined lower limit allows sufficient atmospheric oxygen to reach the catalyst (30) to react with more than 90%, preferably more than 95% and particularly preferably more than 99%, of hydrogen generated at the cathode (24) during use of the oxygen concentrator; and/or optionally, wherein ventilation below the predetermined upper limit limits water carried away from the catalyst (30) by a ventilating airflow during operation of the oxygen concentrator to less than 15%, preferably less than 5%, and particularly preferably less than 1%, of water generated at the catalyst by reaction of hydrogen with atmospheric oxygen;
and/or optionally wherein ventilation below the predetermined upper limit allows more than 85%, preferably more than 95%, and particularly preferably more than 99%, of water generated at the catalyst (30) by reaction of hydrogen with atmospheric oxygen to pass through the diffusion layer (28) to the cathode (24) and the proton-conducting membrane (12).

3. An oxygen concentrator according to claim 1 or 2, wherein the catalyst (30) and the cathode (24) each have area between 150mm² and 2000mm², preferably between 300mm² and 1000mm², and particularly preferably between 400mm² and 600mm²;
and/or wherein the catalyst (30) is substantially planar, and the cathode chamber (14) has a depth, measured perpendicular to the plane of the catalyst, of between 0.4mm and 10mm, preferably between 0.5mm and 7mm, and particularly preferably between 0.6mm and 3mm;
and/or wherein the catalyst (30) is substantially planar and has a lateral dimension, and wherein the cathode chamber (14) has a depth, measured perpendicular to the plane of the catalyst, of between 0.1 and 0.015 times, and preferably of between 0.04 and 0.02 times, the lateral dimension;
and/or wherein the catalyst (30) has a lateral dimension of between 10mm and 50mm.

4. An oxygen concentrator according to any preceding claim, wherein the one or more vents (44) are defined through a wall of the housing (6) and wherein the total area of the one or more vents is between 7mm² and 80mm², preferably between 10mm² and 40mm² and particularly preferably between 12mm² and 20mm²,
and/or wherein the one or more vents (44) are defined through a wall of the housing and wherein the area of the catalyst is between 10 and 70 times, preferably between 25 and 55 times, and particularly preferably between 30 and 45 times, the total area of the one or more vents;
and/or wherein the one or more vents (44) are defined through a wall of the housing and wherein the area of the cathode is between 10 and 70 times, preferably between 25 and 55 times, and particularly preferably between 30 and 45 times, the total area of the one or more vents.

5. An oxygen concentrator according to any preceding claim, configured to operate at a current density between 50Am⁻² and 250Am⁻², preferably between 75Am⁻² and 200Am⁻² and particularly preferably between 100Am⁻² and 150Am⁻²;
and/or configured to operate at a voltage of between 0.75V and 2V, preferably between 1V and 1.5V, and particularly preferably about 1.2V;
or configured to operate at a voltage of between 0.75V and 2V, preferably between 0.8V and 1.2V, and particularly preferably at about 1.0V.

6. An oxygen concentrator according to any preceding claim, configured to produce a flow of oxygen gas from the anode (26) of less than 30ml/hour, measured at atmospheric or ambient pressure, for each 500mm² of the area of the anode.

7. An oxygen concentrator according to any preceding claim, wherein the cathode (24) and/or the anode (26) are in the form of layers coated respectively onto opposite sides of the proton-conducting membrane (12);
and/or wherein the catalyst (30) is in the form of a layer coated onto the diffusion layer (28).

8. An oxygen concentrator according to any preceding claim, wherein the catalyst (30), the diffusion layer (28), the cathode (24), the proton-conducting membrane (12) and the anode (26) are in the form of two or more layers pressed together;
wherein the layers are optionally pressed together between a cathode-side conducting sheet (32) and an anode-side conducting sheet (48) configured to the supply electric current to the layers;
and/or wherein, optionally, the layers are configured to be pressed together by a pressure of more than 0.5MPa, preferably more than 0.8MPa, and particularly preferably more than 0.9MPa.

9. An oxygen concentrator according to any preceding claim, wherein the catalyst (30), the diffusion layer (28), the cathode (24), the proton-conducting membrane (12) and the anode (26) are in the form of two or more layers pressed together by a pressure which is applied by retaining the layers within the housing, stacked together with a compressed layer of a compressible, preferably resilient, material.

10. An oxygen concentrator according to any preceding claim, wherein the oxygen concentrator is configured to produce a continuous oxygen flow of at least 24 l/hr/m² of the area of the catalyst (30), measured at normal temperature and pressure, NTP, when a constant current density is applied to the oxygen concentrator in air of equal to or greater than 35% relative humidity;
wherein the constant current density is optionally less than 150Am⁻² or preferably 120Am⁻², or more preferably about 110Am⁻².

11. An oxygen supply unit comprising an oxygen concentrator (206) as defined in any preceding claim, a power supply (204), such as a rechargeable battery, and an oxygen outlet (210) coupled to an anode side of the oxygen concentrator;
and/or which is wearable or ambulatory and can be coupled to a hyperbaric dressing.

12. An oxygen supply unit according to claim 11, wherein the power supply (204) is configured to supply a predetermined current to the oxygen concentrator (206) and switch to a stand-by condition if the voltage required to drive the predetermined current rises above a predetermined voltage level;
wherein the predetermined current optionally corresponds to a current density across the catalyst or the MEA equal to or less than 150Am⁻² or 120Am⁻², or preferably of about 100Am⁻²;
and/or wherein the predetermined voltage level is optionally 2.0V, or 1.5V, or 1.2V.

13. A method for concentrating oxygen from air, comprising the steps of:
providing a cathode (24) and an anode (26) on opposite sides of a proton-conducting membrane (12), a diffusion layer (28) adjacent to the cathode and a catalyst (30) spaced from the cathode by the diffusion layer;
allowing access of air to the catalyst (30) through one or more vents (44);
passing a current between the cathode (24) and the anode (26) to electrolyse water derived only from humidity in the air and from the catalyst, to produce hydrogen at the cathode and oxygen at the anode; and
reacting the hydrogen with atmospheric oxygen at the catalyst (30) to produce water, to pass through the diffusion layer (28) to the cathode for further electrolysis,
wherein the one or more vents (44) controls the access of air to the catalyst between predetermined upper and lower ventilation rates, and
wherein the cathode is not exposed to atmospheric oxygen.

## Patentansprüche

1. Sauerstoffkonzentrator (2), umfassend;
eine protonenleitende Membran (12);
eine Kathode (24), die eine erste Seite der Membran berührt;
eine Anode (26), die eine zweite Seite der Membran berührt;
eine katalytische Vorrichtung, umfassend einen Katalysator (30) und eine Diffusionsschicht (28), wobei die Diffusionsschicht den Katalysator von der Kathode beabstandet; und
ein Gehäuse (4, 6), das eine Kathodenkammer (14) definiert, wobei die katalytische Vorrichtung innerhalb der Kathodenkammer enthalten ist und das Gehäuse eine oder mehrere Belüftungsöffnungen (44) definiert, die Luft befähigen, zu dem Katalysator zu strömen,
wobei der Sauerstoffkonzentrator kein Wasserreservoir aufweist und konfiguriert ist, um seinen Betrieb durch Elektrolysieren von Wasser, das nur aus Luftfeuchtigkeit und dem Katalysator stammt, und durch Bereitstellung der katalytischen Vorrichtung, die sehr nahe an oder direkt angrenzend an der Kathode positioniert ist, derart aufrechtzuerhalten, dass die katalytische Vorrichtung den von der Kathode erzeugten Wasserstoff in Wasser umwandelt und das Wasser mit geringem Wasserverlust zu der protonenleitenden Membran recycelt, und
wobei die Kathode keinem Luftsauerstoff ausgesetzt ist.

2. Sauerstoffkonzentrator nach Anspruch 1, wobei die eine oder die mehreren Belüftungsöffnungen (44) eine Belüftung der Kathodenkammer (14) unterhalb einer zuvor bestimmten Obergrenze der Belüftungsrate und oberhalb einer zuvor bestimmten Untergrenze der Belüftungsrate befähigen;
optional, wobei Belüftung oberhalb der zuvor bestimmten Untergrenze es ermöglicht, dass genügend Luftsauerstoff den Katalysator (30) erreicht, um mit mehr als 90 %, bevorzugt mehr als 95 % und besonders bevorzugt mehr als 99 % des an der Kathode (24) während einer Verwendung des Sauerstoffkonzentrators erzeugten Wasserstoffs zu reagieren; und/oder optional, wobei Belüftung unterhalb der zuvor bestimmten Obergrenze Wasser, das durch einen Belüftungsluftstrom während des Betriebs des Sauerstoffkonzentrators von dem Katalysator (30) abgeführt wird, auf weniger als 15 %, bevorzugt weniger als 5 % und besonders bevorzugt weniger als 1 % des an dem Katalysator durch Reaktion von Wasserstoff mit Luftsauerstoff erzeugten Wassers begrenzt wird;
und/oder optional wobei Belüftung unterhalb der zuvor bestimmten Obergrenze er mehr als 85 %, bevorzugt mehr als 95 % und besonders bevorzugt mehr als 99 % des an dem Katalysator (30) durch Reaktion von Wasserstoff mit Luftsauerstoff erzeugten Wassers ermöglicht, durch die Diffusionsschicht (28) zu der Kathode (24) und zu der protonenleitenden Membran (12) durchgeleitet zu werden.

3. Sauerstoffkonzentrator nach Anspruch 1 oder 2, wobei der Katalysator (30) und die Kathode (24) jeweils eine Fläche von 150 mm² bis 2000 mm², bevorzugt von 300 mm² bis 1000 mm², und besonders bevorzugt von 400 mm² bis 600 mm² aufweisen;
und/oder wobei der Katalysator (30) im Wesentlichen eben ist und die Kathodenkammer (14) eine Tiefe, senkrecht zu der Ebene des Katalysators gemessen, von 0,4 mm bis 10 mm, bevorzugt von 0,5 mm bis 7 mm und besonders bevorzugt von 0,6 mm bis 3 mm aufweist;
und/oder wobei der Katalysator (30) im Wesentlichen eben ist und eine seitliche Abmessung aufweist, und wobei die Kathodenkammer (14) eine Tiefe, senkrecht zu der Ebene des Katalysators gemessen, des 0,1- bis 0,015-fachen und bevorzugt des 0,04- bis 0,02-fachen der seitlichen Abmessung aufweist;
und/oder wobei der Katalysator (30) eine seitliche Abmessung von 10 mm bis 50 mm aufweist.

4. Sauerstoffkonzentrator nach einem der vorhergehenden Ansprüche, wobei die eine oder die mehreren Belüftungsöffnungen (44) durch eine Wand des Gehäuses (6) definiert sind und wobei die Gesamtfläche der einen oder der mehreren Belüftungsöffnungen von 7 mm² bis 80 mm², bevorzugt von 10 mm² bis 40 mm² und besonders bevorzugt von 12 mm² bis 20 mm² beträgt,
und/oder wobei die eine oder die mehreren Belüftungsöffnungen (44) durch eine Wand des Gehäuses definiert sind und wobei die Fläche des Katalysators das 10- bis 70-fache, bevorzugt das 25- bis 55-fache und besonders bevorzugt das 30- bis 45-fache der Gesamtfläche der einen oder der mehreren Belüftungsöffnungen beträgt;
und/oder wobei die eine oder die mehreren Belüftungsöffnungen (44) durch eine Wand des Gehäuses definiert sind und wobei die Fläche der Kathode das 10- bis 70-fache, bevorzugt das 25- bis 55-fache und besonders bevorzugt das 30- bis 45-fache der Gesamtfläche der einen oder der mehreren Belüftungsöffnungen beträgt.

5. Sauerstoffkonzentrator nach einem der vorhergehenden Ansprüche, der konfiguriert ist, um mit einer Stromdichte von 50 Am⁻² bis 250 Am⁻², bevorzugt von 75 Am⁻² bis 200 Am⁻² und besonders bevorzugt von 100 Am⁻² bis 150 Am⁻² in Betrieb zu sein;
und/oder konfiguriert ist, um bei einer Spannung von 0,75 V bis 2 V, bevorzugt von 1 V bis 1,5 V, und besonders bevorzugt von etwa 1,2 V in Betrieb zu sein;
oder konfiguriert ist, um bei einer Spannung von 0,75 V bis 2 V, bevorzugt von 0,8 V bis 1,2 V, und besonders bevorzugt von etwa 1,0 V in Betrieb zu sein.

6. Sauerstoffkonzentrator nach einem der vorhergehenden Ansprüche, der konfiguriert ist, um einen Sauerstoffgasstrom von der Anode (26) von weniger als 30 ml/Stunde, bei Atmosphären- oder Umgebungsdruck gemessen, für jeweils 500 mm² der Anodenfläche herzustellen.

7. Sauerstoffkonzentrator nach einem der vorhergehenden Ansprüche, wobei die Kathode (24) und/oder die Anode (26) in der Form von Schichten vorliegen, die jeweils auf gegenüberliegenden Seiten der protonenleitenden Membran (12) aufgebracht sind;
und/oder wobei der Katalysator (30) in der Form einer Schicht vorliegt, die auf der Diffusionsschicht (28) aufgebracht ist.

8. Sauerstoffkonzentrator nach einem der vorhergehenden Ansprüche, wobei der Katalysator (30), die Diffusionsschicht (28), die Kathode (24), die protonenleitende Membran (12) und die Anode (26) in der Form von zwei oder mehr zusammengepressten Schichten vorliegen;
wobei die Schichten zwischen einer kathodenseitigen leitenden Platte (32) und einer anodenseitigen leitenden Platte (48) optional zusammengepresst werden, die konfiguriert sind, um die Schichten mit elektrischem Strom zu versorgen;
und/oder wobei optional die Schichten konfiguriert sind, um durch einen Druck von mehr als 0,5 MPa, bevorzugt mehr als 0,8 MPa und besonders bevorzugt mehr als 0,9 MPa zusammengepresst zu werden.

9. Sauerstoffkonzentrator nach einem der vorhergehenden Ansprüche, wobei der Katalysator (30), die Diffusionsschicht (28), die Kathode (24), die protonenleitende Membran (12) und die Anode (26) in der Form von zwei oder mehr Schichten vorliegen, die durch einen Druck zusammengepresst werden, der durch Halten der Schichten innerhalb des Gehäuses angewandt wird, die mit einer komprimierten Schicht aus einem komprimierbaren, bevorzugt elastischen, Material gestapelt sind.

10. Sauerstoffkonzentrator nach einem der vorhergehenden Ansprüche, wobei der Sauerstoffkonzentrator konfiguriert ist, um einen fortlaufenden Sauerstoffstrom von wenigstens 24 l/h/m² der Fläche des Katalysators (30), bei normaler Temperatur und normalem Druck (normal temperature and pressure - NTP) gemessen, zu produzieren, wenn eine konstante Stromdichte an den Sauerstoffkonzentrator in Luft mit einer relativen Feuchtigkeit von über 35 % angewandt wird;
wobei die konstante Stromdichte optional weniger als 150 Am⁻² oder bevorzugt 120 Am⁻², oder stärker bevorzugt etwa 110 Am⁻² beträgt.

11. Sauerstoffversorgungseinheit, umfassend einen Sauerstoffkonzentrator (206), wie in einem der vorhergehenden Ansprüche definiert, eine Stromversorgung (204), wie etwa eine wiederaufladbare Batterie, und einen Sauerstoffauslass (210), der mit einer Anodenseite des Sauerstoffkonzentrators gekoppelt ist;
und/oder die tragbar oder transportabel ist und mit einem hyperbaren Verband verbunden werden kann.

12. Sauerstoffversorgungseinheit nach Anspruch 11, wobei die Stromversorgung (204) konfiguriert ist, um den Sauerstoffkonzentrator (206) mit einem zuvor bestimmten Strom zu versorgen und in einen Bereitschaftszustand zu schalten, falls die Spannung, die benötigt wird, um den zuvor bestimmten Strom anzutreiben, über einen zuvor bestimmten Spannungspegel ansteigt;
wobei der zuvor bestimmte Strom optional einer Stromdichte über dem Katalysator oder der MEA entspricht, die unter 150 Am⁻² oder 120 Am⁻² oder bevorzugt bei etwa 100 Am⁻² liegt;
und/oder wobei der zuvor bestimmte Spannungspegel optional 2,0 V, 1,5 V oder 1,2 V beträgt.

13. Verfahren zum Konzentrieren von Sauerstoff aus der Luft, umfassend die folgenden Schritte:
Bereitstellen einer Kathode (24) und einer Anode (26) auf gegenüberliegenden Seiten einer protonenleitenden Membran (12), einer Diffusionsschicht (28) angrenzend an der Kathode und eines Katalysators (30), der durch die Diffusionsschicht von der Kathode beabstandet ist;
Ermöglichen von Zugang von Luft zu dem Katalysator (30) durch eine oder mehrere Belüftungsöffnungen (44);
Durchleiten eines Stroms zwischen der Kathode (24) und der Anode (26), um Wasser zu elektrolysieren, das nur aus Luftfeuchtigkeit und dem Katalysator stammt, um Wasserstoff an der Kathode und Sauerstoff an der Anode zu produzieren; und
Reagieren des Wasserstoffs mit Luftsauerstoff an dem Katalysator (30), um Wasser zu produzieren, um durch die Diffusionsschicht (28) zu der Kathode für weitere Elektrolyse durchgeleitet zu werden,
wobei die eine oder die mehreren Belüftungsöffnungen (44) den Zugang von Luft zu dem Katalysator zwischen zuvor bestimmten oberen und unteren Belüftungsraten steuert, und
wobei die Kathode keinem Luftsauerstoff ausgesetzt ist.

## Revendications

1. Concentrateur d'oxygène (2) comprenant ;
une membrane conductrice de protons (12) ;
une cathode (24) en contact avec un premier côté de la membrane ;
une anode (26) en contact avec un second côté de la membrane ;
un appareil catalytique comprenant un catalyseur (30) et une couche de diffusion (28), la couche de diffusion espaçant le catalyseur de la cathode ; et
un boîtier (4, 6) définissant une chambre cathodique (14), l'appareil catalytique étant contenu au sein de la chambre cathodique et le boîtier définissant un ou plusieurs évents (44) permettant à l'air de s'écouler vers le catalyseur,
le concentrateur d'oxygène n'ayant pas de réservoir d'eau et étant conçu pour maintenir son fonctionnement en électrolysant de l'eau issue uniquement de l'humidité dans l'air et du catalyseur et en prévoyant que l'appareil catalytique soit positionné très proche de la cathode ou directement à proximité de celle-ci de telle sorte que l'appareil catalytique convertit l'hydrogène généré par la cathode en eau et recycle l'eau vers la membrane conductrice de protons avec une faible perte d'eau, et
la cathode n'étant pas exposée à l'oxygène atmosphérique.

2. Concentrateur d'oxygène selon la revendication 1, le ou les évents (44) permettant la ventilation de la chambre cathodique (14) en dessous d'une limite supérieure prédéterminée de taux de ventilation et au-dessus d'une limite inférieure prédéterminée de taux de ventilation ;
éventuellement la ventilation au-dessus de la limite inférieure prédéterminée permettant à suffisamment d'oxygène atmosphérique d'atteindre le catalyseur (30) pour réagir avec plus de 90 %, de préférence plus de 95 % et de manière particulièrement préférée plus de 99 %, d'hydrogène généré au niveau de la cathode (24) pendant l'utilisation du concentrateur d'oxygène ; et/ou éventuellement, une ventilation en dessous de la limite supérieure prédéterminée limitant l'eau évacuée du catalyseur (30) par un écoulement d'air de ventilation pendant le fonctionnement du concentrateur d'oxygène à moins de 15 %, de préférence à moins de 5 %, et de manière particulièrement préférée à moins de 1 %, d'eau générée au niveau du catalyseur par réaction de l'hydrogène avec l'oxygène atmosphérique ;
et/ou éventuellement une ventilation en dessous de la limite supérieure prédéterminée permettant à plus de 85 %, de préférence plus de 95 %, et de manière particulièrement préférée plus de 99 %, de l'eau générée au niveau du catalyseur (30) par réaction de l'hydrogène avec l'oxygène atmosphérique de passer à travers la couche de diffusion (28) vers la cathode (24) et la membrane conductrice de protons (12).

3. Concentrateur d'oxygène selon la revendication 1 ou 2, le catalyseur (30) et la cathode (24) ayant chacun une surface comprise entre 150 mm² et 2 000 mm², de préférence entre 300 mm² et 1 000 mm², et de manière particulièrement préférée entre 400 mm² et 600 mm² ;
et/ou le catalyseur (30) étant sensiblement plan, et la chambre cathodique (14) ayant une profondeur, mesurée perpendiculairement au plan du catalyseur, comprise entre 0,4 mm et 10 mm, de préférence entre 0,5 mm et 7 mm, et de manière particulièrement préférée entre 0,6 mm et 3 mm ;
et/ou le catalyseur (30) étant sensiblement plan et ayant une dimension latérale, et la chambre cathodique (14) ayant une profondeur, mesurée perpendiculairement au plan du catalyseur, comprise entre 0,1 et 0,015 fois, et de préférence comprise entre 0,04 et 0,02 fois, la dimension latérale ;
et/ou le catalyseur (30) ayant une dimension latérale comprise entre 10 mm et 50 mm.

4. Concentrateur d'oxygène selon l'une quelconque des revendications précédentes, le ou les évents (44) étant définis à travers une paroi du boîtier (6) et la surface totale du ou des évents étant comprise entre 7 mm² et 80 mm², de préférence entre 10 mm² et 40 mm² et de manière particulièrement préférée entre 12 mm² et 20 mm²,
et/ou le ou les évents (44) étant définis à travers une paroi du boîtier et la surface du catalyseur étant comprise entre 10 et 70 fois, de préférence entre 25 et 55 fois, et de manière particulièrement préférée entre 30 et 45 fois, de la surface totale d'un ou plusieurs évents ;
et/ou le ou les évents (44) étant définis à travers une paroi du boîtier et la surface de la cathode étant comprise entre 10 et 70 fois, de préférence entre 25 et 55 fois, et de manière particulièrement préférée entre 30 et 45 fois, de la surface totale d'un ou plusieurs évents.

5. Concentrateur d'oxygène selon l'une quelconque des revendications précédentes, conçu pour fonctionner à une densité de courant comprise entre 50 Am⁻² et 250 Am⁻², de préférence entre 75 Am⁻² et 200 Am⁻² et de manière particulièrement préférée entre 100 Am⁻² et 150 Am⁻² ;
et/ou conçu pour fonctionner à une tension comprise entre 0,75 V et 2 V, de préférence entre 1 V et 1,5 V, et de manière particulièrement préférée environ 1,2 V ;
ou conçu pour fonctionner à une tension comprise entre 0,75 V et 2 V, de préférence entre 0,8 V et 1,2 V, et de manière particulièrement préférée à environ 1,0 V.

6. Concentrateur d'oxygène selon l'une quelconque des revendications précédentes, conçu pour produire un écoulement d'oxygène gazeux depuis l'anode (26) inférieur à 30 ml/heure, mesuré à pression atmosphérique ou ambiante, pour chaque 500 mm² de la surface de l'anode.

7. Concentrateur d'oxygène selon l'une quelconque des revendications précédentes, la cathode (24) et/ou l'anode (26) se trouvant sous la forme de couches appliquées respectivement sur des côtés opposés de la membrane conductrice de protons (12) ;
et/ou le catalyseur (30) se trouvant sous la forme d'une couche appliquée sur la couche de diffusion (28).

8. Concentrateur d'oxygène selon l'une quelconque des revendications précédentes, le catalyseur (30), la couche de diffusion (28), la cathode (24), la membrane conductrice de protons (12) et l'anode (26) se trouvant sous la forme d'au moins deux couches pressées ensemble ;
les couches étant éventuellement pressées ensemble entre une feuille conductrice côté cathode (32) et une feuille conductrice côté anode (48) conçues pour fournir le courant électrique aux couches ;
et/ou, éventuellement, les couches étant conçues pour être pressées ensemble par une pression supérieure à 0,5 MPa, de préférence supérieure à 0,8 MPa, et de manière particulièrement préférée supérieure à 0,9 MPa.

9. Concentrateur d'oxygène selon l'une quelconque des revendications précédentes, le catalyseur (30), la couche de diffusion (28), la cathode (24), la membrane conductrice de protons (12) et l'anode (26) se trouvant sous la forme d'au moins deux couches pressées ensemble par une pression qui est appliquée en retenant les couches au sein du boîtier, empilées ensemble avec une couche comprimée d'un matériau compressible, de préférence élastique.

10. Concentrateur d'oxygène selon l'une quelconque des revendications précédentes, le concentrateur d'oxygène étant conçu pour produire un écoulement d'oxygène continu d'au moins 24 l/h/m² de la surface du catalyseur (30), mesuré à température et pression normales, NTP, lorsqu'une densité de courant constante est appliquée au concentrateur d'oxygène dans l'air égal ou supérieur à 35 % d'humidité relative ;
la densité de courant constante étant éventuellement inférieure à 150 Am⁻² ou de préférence à 120 Am⁻², ou plus préférablement à environ 110 Am⁻².

11. Unité d'approvisionnement en oxygène comprenant un concentrateur d'oxygène (206) tel que défini dans l'une quelconque des revendications précédentes, un approvisionnement électrique (204), telle qu'une batterie rechargeable, et une sortie d'oxygène (210) accouplée au côté anode du concentrateur d'oxygène ;
et/ou qui est portable ou ambulatoire et peut être couplé à un pansement hyperbare.

12. Unité d'approvisionnement en oxygène selon la revendication 11, l'approvisionnement électrique (204) étant conçu pour approvisionner un courant prédéterminé au concentrateur d'oxygène (206) et passer à une condition d'attente si la tension requise pour entraîner le courant prédéterminé s'élève au-dessus d'un niveau de tension prédéterminé ;
le courant prédéterminé correspondant éventuellement à une densité de courant à travers le catalyseur ou le MEA égale ou inférieure à 150 Am⁻² ou 120 Am⁻², ou de préférence d'environ 100 Am⁻² ;
et/ou le niveau de tension prédéterminé étant éventuellement de 2,0 V, ou 1,5 V, ou 1,2 V.

13. Procédé destiné à la concentration d'oxygène de l'air, comprenant les étapes consistant à :
fournir une cathode (24) et une anode (26) sur les côtés opposés d'une membrane conductrice de protons (12), une couche de diffusion (28) à proximité de la cathode et un catalyseur (30) espacé de la cathode par la couche de diffusion ;
permettre l'accès de l'air au catalyseur (30) à travers un ou plusieurs évents (44) ;
faire passer un courant entre la cathode (24) et l'anode (26) pour électrolyser l'eau issue uniquement de l'humidité de l'air et du catalyseur, pour produire de l'hydrogène au niveau de la cathode et de l'oxygène au niveau de l'anode ; et
faire réagir l'hydrogène avec l'oxygène atmosphérique au niveau du catalyseur (30) pour produire de l'eau, pour passer à travers la couche de diffusion (28) jusqu'à la cathode pour une électrolyse supplémentaire,
le ou les évents (44) commandant l'accès de l'air au catalyseur entre des taux de ventilation supérieur et inférieur prédéterminés, et
la cathode n'étant pas exposée à l'oxygène atmosphérique.
